(19) Europäisches Patentamt
European Patent Office
Office européen des brevets

(11) Veröffentlichungsnummer: **0 189 057**
**B1**

(12) # EUROPÄISCHE PATENTSCHRIFT

(45) Veröffentlichungstag der Patentschrift: **21.02.90**

(21) Anmeldenummer: **86100203.8**

(22) Anmeldetag: **09.01.86**

(51) Int. Cl.⁵: **C 07 D 211/90**, C 07 D 211/84, C 07 D 401/04, C 07 D 401/12, C 07 D 401/14, C 07 D 405/04, C 07 D 405/12, C 07 D 405/14, C 07 D 409/04, C 07 D 409/12, C 07 D 409/14

(54) **5-Aryl-dihydropyridine, Verfahren zu ihrer Herstellung sowie ihre Verwendung in Arzneimitteln.**

(30) Priorität: **22.01.85 DE 3501855**

(43) Veröffentlichungstag der Anmeldung: **30.07.86 Patentblatt 86/31**

(45) Bekanntmachung des Hinweises auf die Patenterteilung: **21.02.90 Patentblatt 90/8**

(84) Benannte Vertragsstaaten: **AT BE CH DE FR GB IT LI NL SE**

(56) Entgegenhaltungen:
**EP-A- 0 001 108**
**EP-A- 0 002 208**
**EP-A- 0 071 819**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk (DE)**

(72) Erfinder: **Stoltefuss, Jürgen, Dipl.-Ing., Parkstrasse 20, D-5657 Haan (DE)**
Erfinder: **Gross, Rainer, Dr., Platzhofstrasse 23, D-5600 Wuppertal 1 (DE)**
Erfinder: **Schramm, Matthias, Dr., Paffrather Strasse 38, D-5000 Koeln 80 (DE)**
Erfinder: **Thomas, Günter, Dr., via delle Groane 126, I-20024 Garbagnate Milano (IT)**
Erfinder: **Kayser, Michael, Dr., Fleyerstrasse 231, D-5800 Hagen (DE)**
Erfinder: **Pelster, Bernd, Dr., Pleisufer 6a, D-5205 St. Augustin 1 (DE)**

**EP 0 189 057 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft 5-Aryl-1,4-dihydropyridine, Verfahren zu ihrer Herstellung sowie ihre Verwendung in Arzneimitteln, insbesondere in kreislaufbeeinflussenden Arzneimitteln.

Es ist bereits bekannt, daß bestimmte 1,4-Dihydropyridine interessante pharmakologische Eigenschaften aufweisen [F. Bossert, W. Vater «Die Naturwissenschaften» 58, 578 (1971)]. In der Regel stellen die bekannten wirksamen Verbindungen 1,4-Dihydropyridin-3,5-dicarbonsäureester dar.

Aus EP-A-0 071 819 sind bereits Dihydropyridine mit positiv inotroper Wirkung bekannt. Die erfindungsgemäß ausgewählten Dihydropyridinderivate, die in 5-Position einen Arylsubstituenten tragen, zeigen in unerwarteter Weise eine signifikant stärkere Wirkung, insbesondere bei der Kontraktionskraftverstärkung.

Die Erfindung betrifft neue 5-Aryl-1,4-dihydropyridine der allgemeinen Formel I

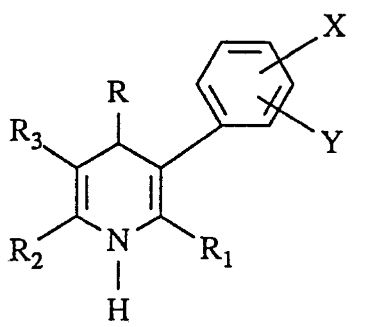

(I)

in welcher

R für $C_4$-$C_7$-Cycloalkyl,

für Thienyl, Pyridyl, Furyl, Benzoxadiazolyl oder

für Phenyl steht, das gegebenenfalls substituiert ist durch bis zu drei gleiche oder verschiedene Substituenten wie Fluor, Chlor, Brom, Nitro, Hydroxy, Carboxy, Cyano, $C_1$-$C_4$-Alkoxycarbonyl Acetyl, Acetyloxy, Benzoyl, Benzoyloxy, gegebenenfalls durch ein oder mehrere Fluoratome substituiertes $C_1$-$C_6$-Alkyl, $C_1$-$C_6$-Alkoxy, $C_1$-$C_6$-Alkylthio oder $C_1$-$C_6$-Alkylsulfonyl, durch gegebenenfalls durch Methyl, Ethyl, Methoxy, Fluor, Chlor oder Nitro substituiertes Phenyl oder gegebenenfalls durch die Gruppe -Z-$CH_2$-$R_4$

wobei

Z für Sauerstoff oder Schwefel steht, und

$R_4$ für $C_4$-$C_7$-Cycloalkyl,

für Thienyl, Furyl oder Pyridyl steht oder

für Phenyl steht, das gegebenenfalls substituiert ist durch Fluor, Chlor, Brom, Cyano, Nitro, Hydroxy, Carboxy, $C_1$-$C_4$-Alkoxycarbonyl oder durch gegebenenfalls durch ein oder mehrere Fluoratome substituiertes $C_1$-$C_6$-Alkyl oder $C_1$-$C_6$-Alkoxy

$R_1$, $R_2$ gleich oder verschieden sind und

für geradkettiges oder verzweigtes $C_1$-$C_5$-Alkyl steht, das gegebenenfalls substituiert ist durch Phenyl, Carboxy, Hydroxy oder $C_1$-$C_4$-Alkoxycarbonyl,

$R_3$ für Nitro, Cyan oder

für die Gruppe -C-A-$R_5$ steht,
‖
O

wobei

A für Sauerstoff steht und wobei

$R_5$ für Wasserstoff,

für $C_4$-$C_7$-Cycloalkyl oder

für einen geradkettigen oder verzweigten, gesättigten oder ungesättigten, gegebenenfalls durch ein bis drei Sauerstoff- und/oder Schwefelatome in der Kette unterbrochenen Kohlenwasserstoffrest mit 1 bis 10 C-Atomen steht, der gegebenenfalls substituiert ist durch ein oder mehrere Fluor-, Chlor-, Bromatome, Nitro-, Cyano-, Hydroxy-, Carboxy-, $C_1$-$C_2$-Alkoxycarbonyl-, Phenyl-, Benzylmethylamino-, Pyridyl-, Furyl- oder Thienylgruppen,

X, Y gleich oder verschieden sind und für Wasserstoff,

für $C_1$-$C_4$-Alkyl, $C_1$-$C_4$-Alkoxy oder $C_1$-$C_4$-Alkoxycarbonyl,

für Fluor, Chlor, Brom, Hydroxy, Nitro, Carboxy, Cyano oder für Trifluormethyl, Trifluormethoxy steht,

sowie deren physiologisch unbedenkliche Salze.

Besonders bevorzugt sind Verbindungen der allgemeinen Formel I, in welcher

R für Cyclophenyl oder Cyclohexyl,

für Thienyl, Furyl oder Pyridyl oder

Phenyl steht, das gegebenenfalls substituiert ist durch ein bis zwei gleiche oder verschiedene Substituenten wie Fluor, Chlor, Nitro, Carboxy, Alkoxycarbonyl (bis 2 C-Atome), Cyan, Phenyl, gegebenenfalls durch ein oder mehrere Fluoratome substituiertes Alkyl, Alkyloxy, Alkylthio oder Alkylsulfonyl (jeweils bis 4 C-Atome), oder gegebenenfalls durch die Gruppe -Z-$CH_2$-$R_4$,

wobei

Z für Sauerstoff oder Schwefel steht und

$R_4$ für Cyclopentyl oder Cyclohexyl,

für Thienyl, Furyl oder Pyridyl oder

für Phenyl steht, das gegebenenfalls substituiert ist durch Fluor, Chlor, Cyano, Nitro, Hydroxy, Carboxy, Alkoxycarbonyl (bis 2 C-Atome) oder durch gegebenenfalls durch ein oder mehrere Fluoratome substituiertes Alkyl oder Alkyloxy (jeweils bis 4 C-Atome),

$R_1$, $R_2$ gleich oder verschieden sind und

für geradkettiges oder verzweigtes Alkyl (bis 4 C-Atome) steht, das gegebenenfalls substituiert ist durch Phenyl, Carboxy oder Alkoxycarbonyl (bis 3 C-Atome),

$R_3$ für Nitro oder

für die Gruppe -C-A-$R_5$ steht,
‖
O

wobei

A für Sauerstoff steht,

$R_5$ für Cyclopentyl, Cyclohexyl oder

für einen geradkettigen oder verzweigten, gesättigten oder ungesättigten, gegebenenfalls durch ein bis zwei Sauerstoff- und/oder Schwefelatome in der Kette unterbrochenen Kohlenwasserstoffrest (bis 8 C-Atome) steht, der gegebenenfalls substituiert ist durch ein oder mehrere Fluor-, Chloratome, Nitro-, Cyano-, Hydroxy- oder Pyridylgruppen,

X, Y gleich oder verschieden sind und

für Wasserstoff,

für Fluor, Chlor, Hydroxy, Nitro, Cyano,

für Trifluormethyl, Trifluormethoxy oder
für Alkyl, Alkoxy, Alkoxycarbonyl (jeweils bis 2 C-Atome) steht,
sowie deren physiologisch unbedenkliche Salze.

Physiologisch unbedenkliche Salze können Salze der erfindungsgemäßen Verbindungen mit anorganischen und organischen Säuren oder Basen sein. Als Beispiele seien genannt: Salze mit Säuren wie Halogenwasserstoffsäuren, Schwefelsäure, Phosphorsäure, Essigsäure, Maleinsäure, Citronensäure, Fumarsäure, Weinsäure, Milchsäure, Benzoesäure sowie Salze mit Basen wie Ammoniak, Alkali- und Erdalkalihydroxiden oder organischen Aminen.

Die erfindungsgemäßen Verbindungen sind neu und besitzen wertvolle pharmakologische Eigenschaften. Sie beeinflussen die Kontraktionskraft des Herzens und können deshalb zur Bekämpfung von cardiovaskulären Erkrankungen verwendet werden. Die neuen 5-Aryl-1,4-dihydropyridine stellen somit eine Bereicherung der Pharmazie dar.

Die erfindungsgemäßen Stoffe der allgemeinen Formel I, in welcher
$R_1$-$R_6$, A, X, Y und Z die oben angegebene Bedeutung haben erhält man, wenn man

(a) Aldehyde der allgemeinen Formel II

R
|
CH  (II)
||
O

in welcher R die oben angegebene Bedeutung hat und Ketoverbindungen der allgemeinen Formel III

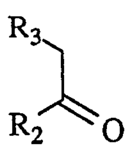

(III)

in welcher $R_2$, $R_3$ die oben angegebene Bedeutung haben mit Ketoverbindungen der allgemeinen Formel IV

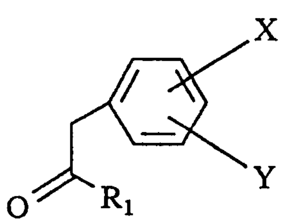

(IV)

in welcher $R_1$, X, Y die oben angegebene Bedeutung haben, und Ammoniak oder einem geeigneten Ammonium-Salz, gegebenenfalls in Anwesenheit von Wasser oder inerten organischen Lösungsmitteln, umsetzt,
oder man

(b) Aldehyde der allgemeinen Formel II mit Ketoverbindungen der allgemeinen Formel III und — gegebenenfalls in situ — hergestellten Enaminen der allgemeinen Formel V

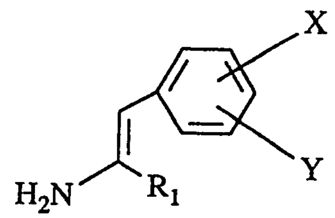

(V)

in welcher $R_1$, X, Y die oben angegebene Bedeutung haben, gegebenenfalls in Anwesenheit von Wasser oder inerten organischen Lösungsmitteln, umsetzt,
oder man

(c) Aldehyde der allgemeinen Formel II mit Ketoverbindungen der allgemeinen Formel IV und Enaminen der allgemeinen Formel VI

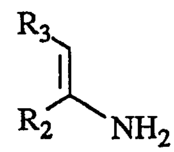

(VI)

in welcher $R_2$, $R_3$ die oben angegebene Bedeutung haben, gegebenenfalls in Anwesenheit von Wasser oder inerten organischen Lösungsmitteln, umsetzt,
oder man

(d) Ylidenverbindungen der allgemeinen Formel VII

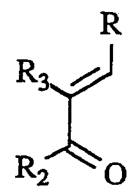

(VII)

in welcher R, $R_2$, $R_3$ die oben angegebenen Bedeutungen haben, mit Ketoverbindungen der allgemeinen Formel IV und Ammoniak oder einem geeigneten Ammoniumsalz, gegebenenfalls in Anwesenheit von Wasser oder inerten organischen Lösungsmitteln, umsetzt,
oder man

(e) Ketoverbindungen der allgemeinen Formel III mit Ylidenverbindungen der allgemeinen Formel VIII

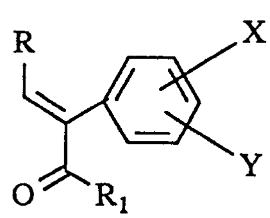

(VIII)

in welcher R, $R_1$, X, Y die oben angegebene Bedeutung haben und Ammoniak oder einem geeigneten Ammoniumsalz, gegebenenfalls in Anwesenheit von Wasser oder inerten organischen Lösungsmitteln, umsetzt,
oder man

(f) Ylidenverbindungen der allgemeinen Formel VII mit Enaminen der allgemeinen Formel V, gegebenenfalls in Anwesenheit von Wasser oder inerten organischen Lösungsmitteln, umsetzt,

oder man

(g) Enamine der allgemeinen Formel VI mit Ylidenverbindungen der allgemeinen Formel VIII, gegebenenfalls in Anwesenheit von Wasser oder inerten organischen Lösungsmitteln, umsetzt.

Je nach Art der verwendeten Ausgangsstoffe kann man die Reaktionen durch folgendes Formelschema darstellen:

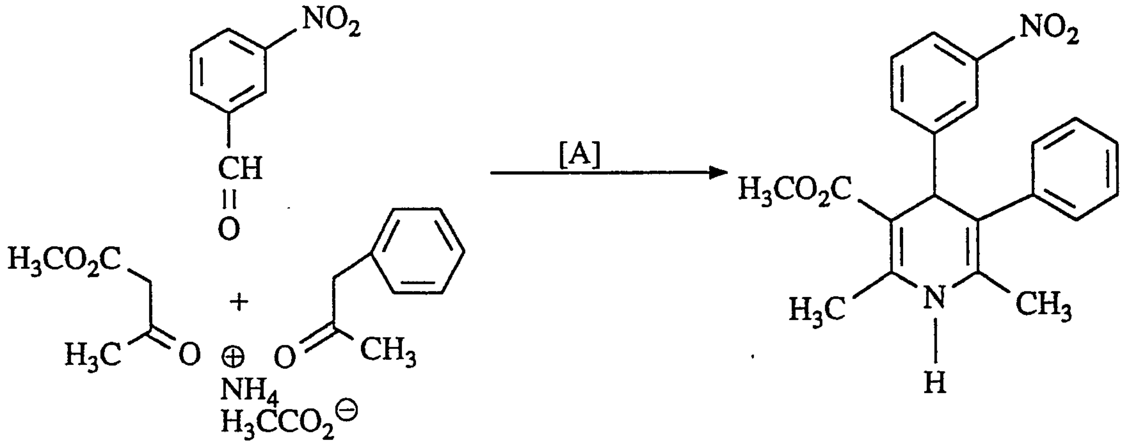


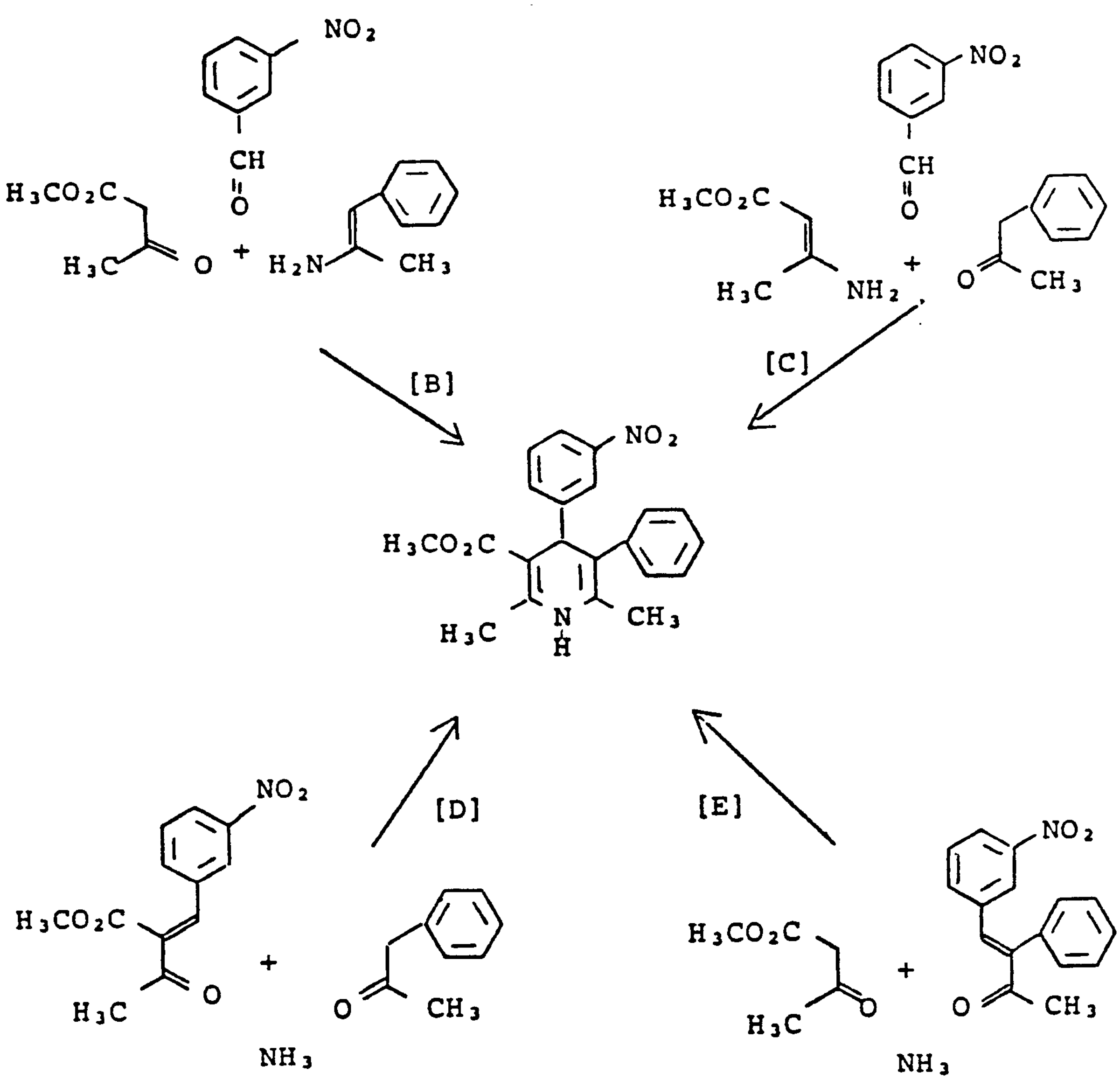

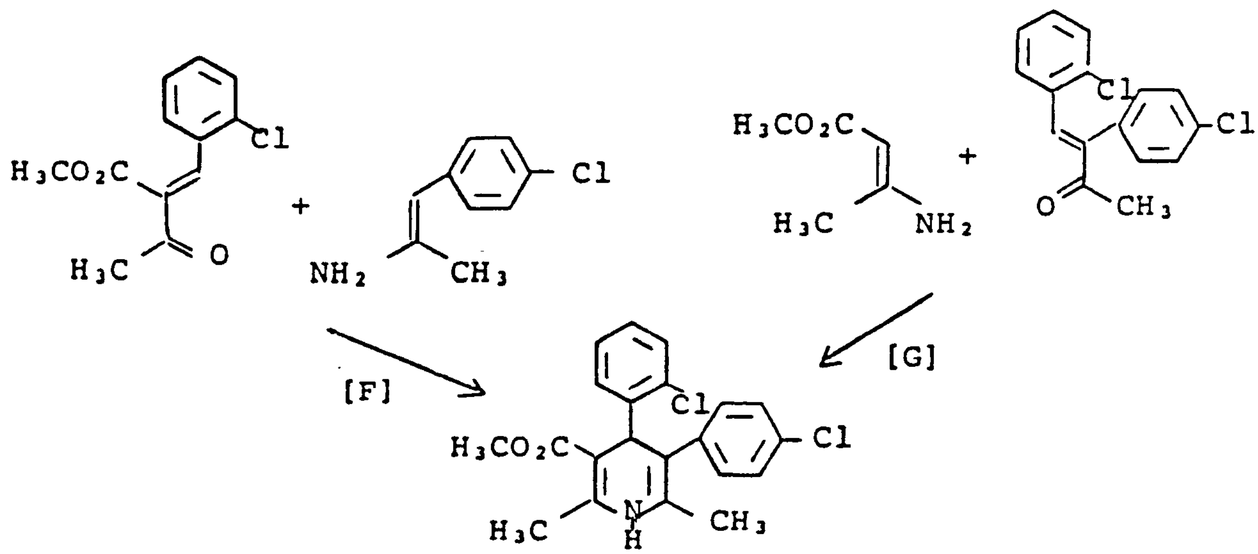


Je nach der Wahl der Ausgangssubstanzen können die erfindungsgemäßen Verbindungen in stereoisomeren Formen existieren, die sich entweder wie Bild und Spiegelbild (Enantiomere) oder die sich nicht wie Bild und Spiegelbild (Diastereomere) verhalten. Sowohl die Antipoden als auch die Racemformen als auch die Diastereomerengemische sind Gegenstand der Erfindung. Die Racemformen lassen sich ebenso wie die Diastereomeren in bekannter Weise in die stereoisomer einheitlichen Bestandteile trennen (vgl. z.B. E.L. Eliel, Stereochemistry of Carbon Compounds, Mc Graw Hill, 1962).

Die verwendeten Aldehyde (II) sind bekannt oder können nach literaturbekannten Methoden dargestellt werden [vgl. T.D. Harris und G.P. Roth, J. Org. Chem. H 2004, 146 (1979); Deutsche Offenlegungsschrift 2 401 665; Mijano et al., Chem. Abstr. 59 (1963), 13 929 c; E. Adler und H.-D. Becker, Acta Chem. Scand. 15, 849 (1961); E.P. Papadopoulos, H.A. Jarrar und C. Issidorides, J. Org. Chem. 31, 615 (1966)].

Die Ketoverbindungen der allgemeinen Formel III sind zum Teil bekannt oder können nach bekannten Methoden hergestellt werden [vgl. N. Levy und C.W. Scaife, J. Chem. Soc. (London) (1946) 1103; C.D. Hurd and M.E. Nilson, J. Org. Chem. 20, 927 (1955); D. Borrmann, «Umsetzung von Diketen mit Alkoholen, Phenolen und Mercaptanen», in Houben-Weyl, Methoden der organischen Chemie, Vol. VII/4, 230 ff. (1968); Y. Oikawa, K. Sugano und O. Yonemitsu, J. Org. Chem. 43, 2087 (1978)].

Die Ketoverbindungen der allgemeinen Formel IV sind bekannt oder können nach bekannten Methoden hergestellt werden [vgl. H.G. Walker, C.R. Hauser, J. Am. Chem. Soc. 68, 1368, (1946); G.G. Smith, J. Am. Chem. Soc. 75, 1134 ff. (1953)].

Die Enamine der allgemeinen Formel V sind bekannt oder können nach bekannten Methoden hergestellt werden [vgl. H. Ahlbrecht, G. Rauchschwalbe, Tetrahedron Letters 51, 4897-4900 (1971)].

Die Enamine der allgemeinen Formel VI sind bekannt oder können nach bekannten Methoden hergestellt werden [vgl. S.A. Glickmann, A.C. Cope, J. Am. Chem. Soc. 67, 1017 (1945); H. Böhme, K.-H. Weisel, Arch. Pharm. 310, 30 (1977)].

Die Ylidenverbindungen der allgemeinen Formel VII sind bekannt oder können nach bekannten Methoden hergestellt werden [vgl. Organic Reactions XV, 204 ff. (1967)].

Die Ylidenverbindungen der allgemeinen Formel VIII sind bekannt oder lassen sich nach bekannten Methoden herstellen [vgl. J.F. Codington, E. Mosettig, J. Org. Chem. 17, 1023 (1952); W. Dilthey, B. Stallmann, Chem. Ber. 62, 1603 (1929)].

Geeignete Ammoniumsalze können Salze von Ammoniak mit anorganischen oder organischen Säuren sein. Es seien beispielhaft aufgeführt: Halogenide, Sulfate, Hydrogensulfate, Hydrogenphosphate, Acetate, Carbonate und Hydrogencarbonate.

Für alle Verfahrensvarianten (a) bis (g) kommen als Verdünnungsmittel Wasser oder alle inerten organischen Lösungsmittel in Frage. Hierzu gehören vorzugsweise Alkohole wie Ethanol, Methanol, Isopropanol, Ether wie Dioxan, Diethylester,Tetrahydrofuran, Glykolmonomethylester, Glykoldimethylester, oder Eisessig, Dimethylformamid, Dimethylsulfoxid, Acetonitril, Pyridin und Hexamethylphosphorsäuretriamid. Bevorzugt wird unter Zusatz von Eisessig gearbeitet.

Die Reaktionstemperaturen können in einem größeren Bereich variiert werden. Im allgemeinen arbeitet man zwischen 0 und 200°C, insbesondere zwischen 10 und 150°C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Die vorstehenden Herstellungsverfahren sind lediglich zur Verdeutlichung angegeben, und die Herstellung der Verbindungen der allgemeinen Formel I ist nicht auf diese Verfahren beschränkt, sondern jede Modifikation dieser Verfahren ist in gleicher Weise für die Herstellung der erfindungsgemäßen Verbindungen anwendbar.

Das Mengenverhältnis der Reaktanden zueinander ist beliebig, im allgemeinen werden jedoch äquimolare Mengen eingesetzt. Es hat sich jedoch als zweckmäßig erwiesen, in Verfahren (e) die Ketoverbindung der allgemeinen Formel III und Ammoniak-Komponente sowie in Verfahren (g) die Enamine der allgemeinen Formel VI im bis zu 10fachen molaren Überschuß einzusetzen.

Die erfindungsgemäßen Verbindungen zeigen ein nicht vorhersehbares, wertvolles pharmakologisches Wirkspektrum. Sie beeinflussen die Kontraktionskraft des Herzens und den Tonus der glatten Muskulatur. Außerdem wirken sie hemmend auf die Lipoxygenase. Sie können deshalb in Arzneimitteln zur Behandlung kardiovaskulärer Erkrankungen Verwendung finden, so z.B. bei der Behandlung von Hypertonie, koronarer Herzkrankheit, Herzinsuffizienz, Hypotonie. Darüberhinaus können sie zur Behandlung von Herzrhythmusstörungen, zur Senkung des Blutzuckers, zur Abschwellung von Schleimhäuten und zur Beeinflussung des Salz- und Flüssigkeitshaushaltes eingesetzt werden.

Die Herz- und Gefäßwirkungen wurden am isoliert perfundierten Herzen des Meerschweinchens gefunden. Dazu werden die Herzen von 250 bis 350 g schweren Albino Meerschweinchen verwendet. Die Tiere werden mit einem Schlag auf den Kopf getötet, der Thorax geöffnet, in die freipräparierte Aorta eine Metallkanüle eingebunden und der linke Vorhof eröffnet. Das Herz wird mit der Lunge aus dem Thorax herausgetrennt und über die Aortenkanüle an die Perfusionsapparatur bei laufender Perfusion angeschlossen.

Die Lunge wird an den Lungenwurzeln abgetrennt. Als Perfusionsmedium dient Krebs-Henseleit-Lösung (118,5 mmol/l NaCl, 4,75 mmol/l KCl, 1,19 mmol/l $KH_2PO_4$, 119 mmol/l $MgSO_4$, 25 mmol/l $NaHCO_3$, 0,013 mmol/l NaEDTA), deren $CaCl_2$ je nach Bedarf variiert wird, in der Regel jedoch 1,2 mmol/l beträgt. Als energielieferndes Substrat werden 10 mmol/l Glucose zugesetzt. Vor der Perfusion wird die Lösung partikelfrei filtriert. Die Lösung wird mit Carbogen (95% $O_2$, 5% $CO_2$) zur Aufrechterhaltung des pH-Wertes von 7,4 begast. Die Herzen werden mit konstantem Fluß (10 ml/min) bei 32°C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt und die isovolumetrischen Kontraktionen auf einem Schnellschreiber registriert [Opie, L., J. Physiol. 180 (1965) 529-541]. Der Perfusionsdruck wird mittels eines Druckaufnehmers, der vor dem Herzen mit dem Perfusionssystem in Verbindung steht, registriert. Unter diesen Bedingungen zeigt eine Senkung des Perfusionsdruckes eine Koronardilation, eine Steigerung der linksventrikulären Druckamplitude einen Anstieg der Herzkontraktilität an. Die erfindungsgemäßen Verbindungen werden in geeigneten Verdünnungen in das Perfusionssystem kurz vor dem isolierten Herzen infundiert.

| Beispiel Nr. | Änderung der Kontraktionsamplitude ($10^{-7}$ g/ml Substanz) |
|---|---|
| 1 | + 37% |
| 19 | + 22% |

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester), Polyoxyethylen-Fettalkohol-Ether (z.B. Lignin, Sulfitablaugen, Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspension und/oder Elixieren, die für orale Anwendung gedacht sind, können die Wirkstoffe außer den obengenannten Hilfsstoffen mit verschiedenen Geschmackaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg, Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg, Körpergewicht.

Trotzdem kann es gegebenenfalls erforderlich sein, von den genannten Mengen abzuweichen, und zwar in Abhängigkeit vom Körpergewicht des Versuchstieres bzw. der Art der Applikation, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenwert sein, diese in mehrere Einzelgaben über den Tag zu verteilen. Für die Applikation in der Humanmedizin ist der gleiche Dosierungspielraum vorgesehen. Sinngemäß gelten hierbei auch die obigen Ausführungen.

*Herstellungsbeispiele*

*Beispiel 1*

4-(2-Benzyloxyphenyl)-1,4-dihydro-2,6-dimethyl-3-nitro-5-phenylpyridin

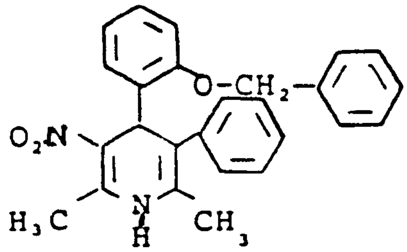

3 g (10 mmol) 2-Benzyloxy-benzylidene-nitroaceton werden in 15 ml Ethanol mit 1,34 g (10 mmol) Phenylaceton und 770 mg (10 mmol) Ammoniumacetat unter Rückfluß 1,5 Stunden erhitzt. Es wird abgekühlt und eingeengt. Der Eindampfrückstand wird in Essigester aufgenommen, mit Wasser gewaschen, getrocknet und eingeengt. Der erhaltene Eindampfrückstand wird über eine Säule von 150 ml Volumen, stationärer Phase Kieselgel und mobiler Phase Toluol/Essigester 10 : 1 gereinigt. Die sauberen Fraktionen werden vereint, eingeengt und mit Ether kristallisiert. Man erhält 0,2 g gelber Kristalle vom Schmelzpunkt 189°C.

*Beispiel 2*

4-(3-Chlorphenyl)-1,4-dihydro-2,6-dimethyl-5-phenylpyridin-3-carbonsäuremethylester

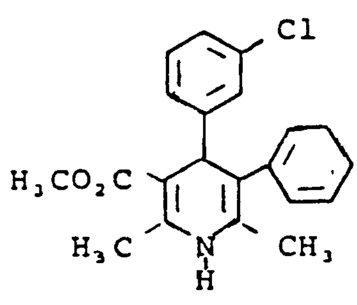

12,82 g (50 mmol) 1-(3-Chlorphenyl)-2-phenyl-3-oxobuten-(1) werden in 80 ml Ethanol mit 11,5 g (100 mmol) β-Aminocrotonsäuremethylester und 6 ml (100 mmol) Essigsäure über Nacht zum Rückfluß erhitzt. Es werden noch einmal 11,5 g β-Aminocrotonsäuremethylester und 6 ml Essigsäure zugefügt und 24 Stunden unter Rückfluß erhitzt. Beim Abkühlen fallen Kristalle an, die abgesaugt und mit Ethanol gewaschen werden. Zur völligen Reinigung wird über eine Kieselgelsäule mit Toluol/Essigester 20 : 1 gegeben. Man erhält 5,3 g einer farblosen Substanz vom Schmelzpunkt 191 °C.

*Beispiel 3*

4-(3-Chlorphenyl)-1,4-dihydro-2,6-dimethyl-3-nitro-5-phenyl-pyridin

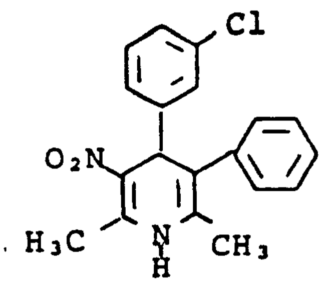

12,82 g (50 mmol) 1-(3-Chlorphenyl)-2-phenyl-3-oxobuten-(1) werden in 75 ml Ethanol mit 5,15 g (50 mmol) Nitroaceton und 3,85 g (50 mmol) Ammoniumacetat 16 Stunden unter Rückfluß erhitzt. Es werden noch 5,15 g Nitroaceton und 3,85 g Ammoniumacetat zugegeben, 24 Stunden gekocht, 5,15 g Nitroaceton und 3,85 g Ammoniumacetat zugefügt und 24 Stunden gekocht. Es wird abgekühlt, eingeengt, in Essigester aufgenommen, 2mal mit Wasser ausgeschüttelt, getrocknet und eingeengt.

Reinigung über Kieselgelsäule mit Toluol/Essigester 10 : 1. Die sauberen Fraktionen werden gesammelt, eingeengt, mit Ether verrührt, abgesaugt und mit Ether gewaschen. Man erhält 5,2 g orangefarbener Kristalle vom Schmelzpunkt 157-60°C.

*Beispiel 4*

4-(2-Chlorphenyl)-1,4-dihydro-2,6-dimethyl-3-nitro-5-phenyl-pyridin

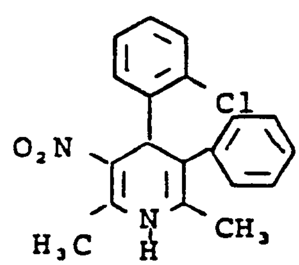

1,5 g (10 mmol) 2-Chlorbenzaldehyd werden mit 1,03 g (10 mmol) Nitroaceton, 2,7 g (20 mmol) Phenylaceton und 0,7 g (10 mmol) Ammoniumacetat in 20 ml Ethanol über Nacht gekocht. Es wird eingeengt, in Essigester aufgenommen, mit Wasser gewaschen, getrocknet und eingeengt. Der Eindampfrückstand wird über eine Kieselgelsäule mit Toluol/Essigester 10 : 1 gereinigt. Die Produkt enthaltenden Fraktionen werden gesammelt und eingeengt. Die Substanz kristallisiert mit Ether. Es wird abgesaugt und mit Ether gewaschen. Man erhält 200 mg orangefarbener Kristalle vom Schmelzpunkt 232°C.

Die folgenden Stoffe werden analog den vorstehenden, in den Beispielen 1 bis 4 aufgezeigten, Verfahren erhalten:

*Beispiel 5*
4-(2-Benzyloxyphenyl)-5-(4-cyanophenyl)-1,4-dihydro-2,6-dimethyl-3-nitro-pyridin
Schmelzpunkt: 126°C.

*Beispiel 6*
4-(3-Chlorphenyl)-1,4-dihydro-5-(4-methoxyphenyl)-2,6-dimethyl-pyridin-5-carbonsäuremethylester
Schmelzpunkt: 171°C.

*Beispiel 7*
6-Benzyl-4-(2-benzyloxyphenyl)-1,4-dihydro-2--methyl-3-nitro-5-phenyl-pyridin
Schmelzpunkt: 173°C.

*Beispiel 8*
1,4-Dihydro-2,6-dimethyl-3-nitro-4,5-diphenyl-pyridin
Schmelzpunkt: 187°C.

*Beispiel 9*
6-Benzyl-4-(2-chlorphenyl)-1,4-dihydro-2-methyl--3-nitro-5-phenyl-pyridin
Schmelzpunkt: 177°C.

*Beispiel 10*
1,4-Dihydro-2,6-dimethyl-3-nitro-4-(2-nitrophenyl)-5-phenyl-pyridin
Schmelzpunkt: 166-170°C.

*Beispiel 11*
6-Benzyl-1,4-dihydro-2-methyl-3-nitro-4,5-diphenyl-pyridin
Schmelzpunkt: 218°C.

*Beispiel 12*
4-(2-Chlorphenyl)-1,4-dihydro-2,6-dimethyl-5--phenyl-pyridin-3-carbonsäuremethylester
Schmelzpunkt: 130°C.

*Beispiel 13*
4-(2-Chlorphenyl)-1,4-dihydro-2,6-dimethyl-5--phenyl-pyridin-3-carbonsäuremethylester
Schmelzpunkt: 108°C.

*Beispiel 14*
4-(2-Fluorphenyl)-1,4-dihydro-2,6-dimethyl-5--phenyl-3-nitro-pyridin
Schmelzpunkt: 206°C.

*Beispiel 15*
4-(2-Chlorphenyl)-1,4-dihydro-2-methoxy-carbonylmethyl-6-methyl-5-phenyl-pyridin-3-carbonsäuremethylester
Schmelzpunkt: 163°C.

*Beispiel 16*
6-Benzyl-1,4-dihydro-2-methyl-4,5-diphenyl-pyridin-3-carbonsäuremethylester
Schmelzpunkt: 184°C.

*Beispiel 17*
4-(2-Chlorphenyl)-6-ethyl-1,4-dihydro-2-methyl--3-nitro-5-phenyl-pyridin
Schmelzpunkt: 195°C.

*Beispiel 18*
1,4-Dihydro-2,6-dimethyl-3-nitro-5-phenyl-4-(2--trifluormethylphenyl)-pyridin
Schmelzpunkt: 206°C.

*Beispiel 19*
1,4-Dihydro-2,6-dimethyl-3-nitro-4-(3-nitrophenyl)-5-phenyl-pyridin
Schmelzpunkt: 157°C.

*Beispiel 20*
1,4-Dihydro-2,6-dimethyl-4-(3-nitrophenyl)-5--phenyl-3-carbonsäuremethylester
Schmelzpunkt: 152°C.

*Beispiel 21*
1,4-Dihydro-2,6-dimethyl-4-(2-nitrophenyl)-5--phenyl-pyridin-3-carbonsäuremethylester
Schmelzpunkt: 180°C.

*Beispiel 22*
4-(3-Chlorphenyl)-2-ethoxycarbonylmethyl-1,4-dihydro-6-methyl-5-phenyl-pyridin-3-carbonsäureethylester
Schmelzpunkt: 129-132°C.

*Beispiel 23*
1,4-dihydro-2,6-dimethyl-3-nitro-5-phenyl-4-(3--trifluoro-phenyl)-pyridin
Schmelzpunkt: 162°C.

*Beispiel 24*
1,4-Dihydro-2,6-dimethyl-3-nitro-5-phenyl-4-(4--trifluor-methylmercapto-phenyl)-pyridin
Schmelzpunkt: 162°C.

*Beispiel 25*
4-Cyclohexyl-1,4-dihydro-2,6-dimethyl-3-nitro-5--phenyl-pyridin
Schmelzpunkt: 195-97°C.

*Beispiel 26*
4,5-Bis-(4-chlorphenyl)-1,4-dihydro-2,6-dimethyl--3-nitro-pyridin
Schmelzpunkt: 115-116°C.

*Beispiel 27*
1,4-Dihydro-2,6-dimethyl-3-nitro-5-phenyl-4-thienyl-pyridin
Schmelzpunkt: 157-58°C.

*Beispiel 28*
1,4-Dihydro-4-(4-methoxyphenyl)-2,6-dimethyl-5--nitro-3-phenyl-pyridin
Schmelzpunkt: 181°C.

*Beispiel 29*
1,4-Dihydro-2,6-dimethyl-5-phenyl-4-(2-trifluormethyl-phenyl)-pyridin-3-carbonsäuremethylester
$R_f$-Wert: 0,51
DC Alurolle Merck, Fließmittel Toluol/Essigester im Volumenverhältnis 4 : 1.
Schmelzpunkt: 143°C.

*Beispiel 30*

4-(4-Hydroxy-3-methoxyphenyl)-1,4-dihydro-2,6--dimethyl-5-phenyl-3-nitro-pyridin

$R_f$-Wert: 0,1

Schmelzpunkt:r 227°C.

*Beispiel 31*

4-(4-Hydroxyphenyl)-1,4-dihydro-2,6-dimethyl-3--nitro-5-phenyl-pyridin

$R_f$-Wert: 0,095

Schmelzpunkt: 240°C.

*Beispiel 32*

1,4-Dihydro-4-(4-hydroxyphenyl)-2,6-dimethyl--5-phenyl-pyridin-3-carbonsäuremethylester

$R_f$-Wert: 0,66 Fließmittel Toluol/Essigester im Volumenverhältnis 1 : 1.

*Beispiel 33*

1,4-Dihydro-2,6-dimethyl-4-(2-fluormethyl)-5-(4--methoxy-phenyl)-3-nitro-pyridin

Schmelzpunkt: ab 297°C.

*Beispiel 34*

4-(2-Chlorphenyl)-5-(4-cyanophenyl)-1,4-dihydro-2,6-dimethyl-3-nitro-pyridin

Schmelzpunkt: 213°C.

**Patentansprüche**

1. 5-Aryl-1,4-dihydropyridine der allgemeinen Formel I

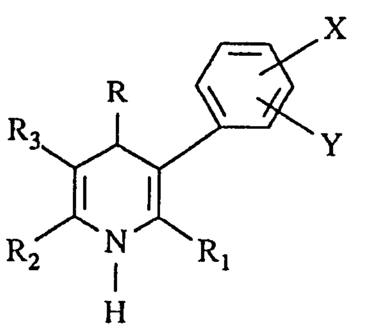

(I)

in welcher

R für $C_4$-$C_7$-Cycloalkyl,

für Thienyl, Pyridyl, Furyl, Benzoxadiazolyl oder

für Phenyl steht, das gegebenenfalls substituiert ist durch bis zu drei gleiche oder verschiedene Substituenten wie Fluor, Chlor, Brom, Nitro, Hydroxy, Carboxy, Cyano, $C_1$-$C_4$-Alkoxycarbonyl Acetyl, Acetyloxy, Benzoyl, Benzoyloxy, gegebenenfalls durch ein oder mehrere Fluoratome substituiertes $C_1$-$C_6$-Alkyl, $C_1$-$C_6$-Alkoxy, $C_1$-$C_6$-Alkylthio oder $C_1$-$C_6$-Alkylsulfonyl, durch gegebenenfalls durch Methyl, Ethyl, Methoxy, Fluor, Chlor oder Nitro substituiertes Phenyl oder gegebenenfalls durch die Gruppe -Z-$CH_2$-$R_4$

wobei

Z für Sauerstoff oder Schwefel steht, und

$R_4$ für $C_4$-$C_7$-Cycloalkyl,

für Thienyl, Furyl oder Pyridyl steht oder

für Phenyl steht, das gegebenenfalls substituiert ist durch Fluor, Chlor, Brom, Cyano, Nitro, Hydroxy, Carboxy, $C_1$-$C_4$-Alkoxycarbonyl oder durch gegebenenfalls durch ein oder mehrere Fluoratome substituiertes $C_1$-$C_6$-Alkyl oder $C_1$-$C_6$-Alkoxy

$R_1$, $R_2$ gleich oder verschieden sind und

für geradkettiges oder verzweigtes $C_1$-$C_5$-Alkyl steht, das gegebenenfalls substituiert ist durch Phenyl, Carboxy, Hydroxy oder $C_1$-$C_4$-Alkoxycarbonyl,

$R_3$ für Nitro, Cyan oder

für die Gruppe $\underset{\underset{O}{\|}}{-C}-A-R_5$ steht,

wobei

A für Sauerstoff steht und wobei

$R_5$ für Wasserstoff

für $C_4$-$C_7$-Cycloalkyl oder

für einen geradkettigen oder verzweigten, gesättigten oder ungesättigten, gegebenenfalls durch ein bis drei Sauerstoff- und/oder Schwefelatome in der Kette unterbrochenen Kohlenwasserstoffrest mit 1 bis 10 C-Atomen steht, der gegebenenfalls substituiert ist durch ein oder mehrere Fluor-, Chlor-, Bromatome, Nitro-, Cyano-, Hydroxy-, Carboxy-, $C_1$-$C_2$-Alkoxycarbonyl-, Phenyl-, Benzylmethylamino-, Pyridyl-, Furyl- oder Thienylgruppen,

X, Y gleich oder verschieden sind und für Wasserstoff,

für $C_1$-$C_4$-Alkyl, $C_1$-$C_4$-Alkoxy oder $C_1$-$C_4$-Alkoxycarbonyl,

für Fluor, Chlor, Brom, Hydroxy, Nitro, Carboxy, Cyano oder

für Trifluormethyl, Trifluormethoxy steht,

sowie deren physiologisch unbedenkliche Salze.

2. 5-Aryl-1,4-dihydropyridine der allgemeinen Formel I in Anspruch 1, in welcher

R für Cyclophenyl oder Cyclohexyl,

für Thienyl, Furyl oder Pyridyl oder

Phenyl steht, das gegebenenfalls substituiert ist durch ein bis zwei gleiche oder verschiedene Substituenten wie Fluor, Chlor, Nitro, Carboxy, $C_1$-$C_2$-Alkoxycarbonyl, Cyan, Phenyl, gegebenenfalls durch ein oder mehrere Fluoratome substituiertes $C_1$-$C_4$-Alkyl, $C_1$-$C_4$-Alkyloxy, $C_1$-$C_4$-Alkylthio oder $C_1$-$C_4$-Alkylsulfonyl, oder gegebenenfalls durch die Gruppe -Z-$CH_2$-$R_4$,

wobei

Z für Sauerstoff oder Schwefel steht und

$R_4$ für Cyclopentyl oder Cyclohexyl,

für Thienyl, Furyl oder Pyridyl oder

für Phenyl steht, das gegebenenfalls substituiert ist durch Fluor, Chlor, Cyano, Nitro, Hydroxy, Carboxy, $C_1$-$C_2$-Alkoxycarbonyl oder durch gegebenenfalls durch ein oder mehrere Fluoratome substituiertes $C_1$-$C_4$-Alkyl oder $C_1$-$C_4$-Alkyloxy,

$R_1$, $R_2$ gleich oder verschieden sind und

für geradkettiges oder verzweigtes $C_1$-$C_4$-Alkyl steht, das gegebenenfalls substituiert ist durch Phenyl, Carboxy oder $C_1$-$C_3$-Alkoxycarbonyl,

$R_3$ für Nitro oder

für die Gruppe $\underset{\underset{O}{\|}}{-C}-A-R_5$ steht,

wobei

A für Sauerstoff steht,

$R_5$ für Cyclopentyl, Cyclohexyl oder

für einen geradkettigen oder verzweigten, gesättigten oder ungesättigten, gegebenenfalls durch ein bis zwei Sauerstoff- und/oder Schwefelatome in der Kette unterbrochenen Kohlenwasserstoffrest mit bis zu 8 C-Atomen steht, der gegebenenfalls substituiert ist durch ein oder mehrere Fluor-, Chloratome, Nitro-, Cyano-, Hydroxy- oder Pyridylgruppen,

X, Y gleich oder verschieden sind und

für Wasserstoff,

für Fluor, Chlor, Hydroxy, Nitro, Cyano,

für Trifluormethyl, Trifluormethoxy oder

für $C_1$-$C_2$-Alkyl, $C_1$-$C_2$-Alkoxy, $C_1$-$C_2$-Alkoxycarbonyl steht,

sowie deren physiologisch unbedenkliche Salze.

3. Verbindungen gemäss Ansprüche 1 bis 2, zur Bekämpfung von Herzinsuffizienz, Hypertonie, Herzrhythmusstörungen sowie zur Abschwellung von Schleimhäuten, Senkungen des Blutzuckers und Beeinflussung des Salz- und Flüssigkeithaushaltes.

4. Arzneimittel enthatend als Wirkstoff Verbindungen gemäss Ansprüche 1 bis 2.

5. Verfahren zur Herstellung von Arzneimitteln gemäß Anspruch 4, dadurch gekennzeichnet, daß man den Wirkstoff mit üblichen Hilfs- und Trägerstoffen mischt.

6. Verfahren zur Herstellung von 5-Aryl-1,4-dihydro-pyridinen der allgemeinen Formel I

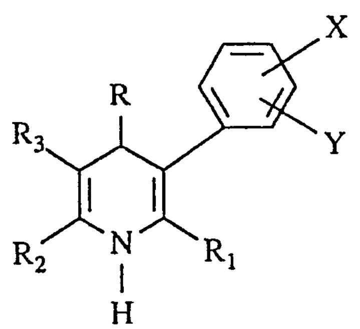 (I)

in welcher

R für $C_4$-$C_7$-Cycloalkyl,

für Thienyl, Pyridyl, Furyl, Benzoxadiazolyl oder

für Phenyl steht, das gegebenenfalls substituiert ist durch bis zu drei gleiche oder verschiedene Substituenten wie Fluor, Chlor, Brom, Nitro, Hydroxy, Carboxy, Cyano, $C_1$-$C_4$-Alkoxycarbonyl Acetyl, Acetyloxy, Benzoyl, Benzoyloxy, gegebenenfalls durch ein oder mehrere Fluoratome substituiertes $C_1$-$C_6$-Alkyl, $C_1$-$C_6$-Alkoxy, $C_1$-$C_6$-Alkylthio oder $C_1$-$C_6$-Alkylsulfonyl, durch gegebenenfalls durch Methyl, Ethyl, Methoxy, Fluor, Chlor oder Nitro substituiertes Phenyl oder gegebenenfalls durch die Gruppe $-Z-CH_2-R_4$

wobei

Z für Sauerstoff oder Schwefel steht, und

$R_4$ für $C_4$-$C_7$-Cycloalkyl,

für Thienyl, Furyl oder Pyridyl steht oder

für Phenyl steht, das gegebenenfalls substituiert ist durch Fluor, Chlor, Brom, Cyano, Nitro, Hydroxy, Carboxy, $C_1$-$C_4$-Alkoxycarbonyl oder durch gegebenenfalls durch ein oder mehrere Fluoratome substituiertes $C_1$-$C_6$-Alkyl oder $C_1$-$C_6$-Alkoxy,

$R_1$, $R_2$ gleich oder verschieden sind und

für geradkettiges oder verzweigtes $C_1$-$C_5$-Alkyl steht, das gegebenenfalls substituiert ist durch Phenyl, Carboxy, Hydroxy oder $C_1$-$C_4$-Alkoxycarbonyl,

$R_3$ für Nitro, Cyan oder

für die Gruppe -C-A-$R_5$ steht,
‖
O

wobei

A für Sauerstoff steht und wobei

$R_5$ für Wasserstoff,

für $C_4$-$C_7$-Cycloalkyl oder

für einen geradkettigen oder verzweigten, gesättigten oder ungesättigten, gegebenenfalls durch ein bis drei Sauerstoff- und/oder Schwefelatome in der Kette unterbrochenen Kohlenwasserstoffrest mit 1 bis 10 C-Atomen steht, der gegebenenfalls substituiert ist durch ein oder mehrere Fluor-, Chlor-, Bromatome, Nitro-, Cyano-, Hydroxy-, Carboxy-, $C_1$-$C_2$-Alkoxycarbonyl-, Phenyl-, Benzylmethylamino-, Pyridyl-, Furyl- oder Thienylgruppen,

X, Y gleich oder verschieden sind und für Wasserstoff,

für $C_1$-$C_4$-Alkyl, $C_1$-$C_4$-Alkoxy oder $C_1$-$C_4$-Alkoxycarbonyl,

für Fluor, Chlor, Brom, Hydroxy, Nitro, Carboxy, Cyano oder

für Trifluormethyl, Trifluormethoxy steht,

dadurch gekennzeichnet, daß man

(a) Aldehyde der allgemeinen Formel II

R
I
CH (II)
‖
O

in welcher R die oben angegebene Bedeutung hat und Ketoverbindungen der allgemeinen Formel III

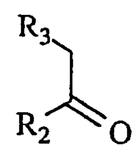 (III)

in welcher $R_2$, $R_3$ die oben angegebene Bedeutung haben mit Ketoverbindungen der allgemeinen Formel IV

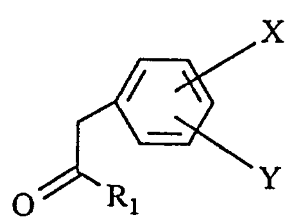 (IV)

in welcher $R_1$, X, Y die oben angegebene Bedeutung haben, und Ammoniak oder einem geeigneten Ammonium-Salz, gegebenenfalls in Anwesenheit von Wasser oder inerten organischen Lösungsmitteln, umsetzt,

oder man

(b) Aldehyde der allgemeinen Formel II mit Ketoverbindungen der allgemeinen Formel III und — gegebenenfalls in situ — hergestellten Enaminen der allgemeinen Formel V

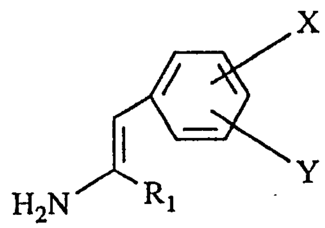

(V)

in welcher $R_1$, X, Y die oben angegebene Bedeutung haben, gegebenenfalls in Anwesenheit von Wasser oder inerten organischen Lösungsmitteln, umsetzt,

oder man

(c) Aldehyde der allgemeinen Formel II mit Ketoverbindungen der allgemeinen Formel IV und Enaminen der allgemeinen Formel VI

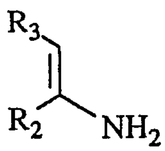

(VI)

in welcher $R_2$, $R_3$ die oben angegebene Bedeutung haben, gegebenenfalls in Anwesenheit von Wasser oder inerten organischen Lösungsmitteln, umsetzt,

oder man

(d) Ylidenverbindungen der allgemeinen Formel VII

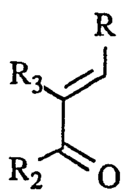

(VII)

in welcher R, $R_2$, $R_3$ die oben angegebenen Bedeutungen haben, mit Ketoverbindungen der allgemeinen Formel IV und Ammoniak oder einem geeigneten Ammoniumsalz, gegebenenfalls in Anwesenheit von Wasser oder inerten organischen Lösungsmitteln, umsetzt,

oder man

(e) Ketoverbindungen der allgemeinen Formel III mit Ylidenverbindungen der allgemeinen Formel VIII

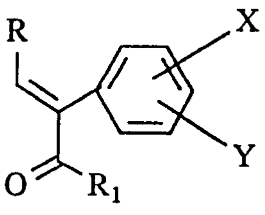

(VIII)

in welcher R, $R_1$, X, Y die oben angegebene Bedeutung haben und Ammoniak oder einem geeigneten Ammoniumsalz, gegebenenfalls in Anwesenheit von Wasser oder inerten organischen Lösungsmitteln, umsetzt,

oder man

(f) Ylidenverbindungen der allgemeinen Formel VII mit Enaminen der allgemeinen Formel V, gegebenenfalls in Anwesenheit von Wasser oder inerten organischen Lösungsmitteln, umsetzt,

oder man

(g) Enamine der allgemeinen Formel VI mit Ylidenverbindungen der allgemeinen Formel VIII, gegebenenfalls in Anwesenheit von Wasser oder inerten organischen Lösungsmitteln, umsetzt.

7. Verfahren gemäß Anspruch 6, dadurch gekennzeichnet, daß man die Reaktionen bei 0 bis 200°C durchführt.

8. Verfahren gemäß Ansprüchen 6 und 7, dadurch gekennzeichnet, daß man 5-Aryl-1,4-dihydropyridine der allgemeinen Formel I herstellt mit folgenden Substituentendefinitionen:

R Cyclopentyl oder Cyclohexyl,

Thienyl, Furyl, oder Pyridyl oder

Phenyl, das gegebenenfalls substituiert ist durch ein bis zu zwei gleiche oder verschiedene Substituenten wie Fluor, Chlor, Brom, Nitro, Carboxy, $C_1$-$C_2$-Alkoxycarbonyl, Cyan, Phenyl, gegebenenfalls durch ein oder mehrere Fluoratome substituiertes $C_1$-$C_4$-Alkyl, $C_1$-$C_4$-Alkyloxy, $C_1$-$C_4$-Alkylthio oder $C_1$-$C_4$-Alkylsulfonyl oder gegebenenfalls durch die Gruppe $-Z-CH_2-R_4$

wobei

Z für Sauerstoff oder Schwefel steht und

$R_4$ für $C_4$-$C_7$-Cyclopentyl oder Cyclohexyl,

für Thienyl, Furyl oder Pyridyl oder

für Phenyl steht, das gegebenenfalls substituiert ist durch Fluor, Chlor, Cyano, Nitro, Hydroxy, Carboxy, $C_1$-$C_2$-Alkoxycarbonyl oder durch gegebenenfalls durch ein oder mehrere Fluoratome substituiertes $C_1$-$C_4$-Alkyl oder $C_1$-$C_4$-Alkyloxy,

$R_1$, $R_2$ gleich oder verschieden,

geradkettiges oder verzweigtes $C_1$-$C_4$-Alkyl, das gegebenenfalls substituiert ist durch Phenyl, Carboxy oder $C_1$-$C_3$-Alkoxycarbonyl,

$R_3$ Nitro oder

die Gruppe $-\underset{\underset{O}{\|}}{C}-A-R_5$,

wobei

A für Sauerstoff steht,

$R_5$ für Cyclopentyl, Cyclohexyl oder

für einen geradkettigen oder verzweigten, gesättigten oder ungesättigten, gegebenenfalls durch ein bis zwei Sauerstoff- und/oder Schwefelatome in der Kette unterbrochenen Kohlenwasserstoffrest mit bis zu 8 C-Atomen steht, der gegebenenfalls substituiert ist durch ein oder mehrere Fluor-, Chloratome, Nitro-, Cyano-, Hydroxy- oder Pyridylgruppen,

X, Y gleich oder verschieden,

Wasserstoff,

Fluor, Chlor, Hydroxy, Nitro, Cyano,

Trifluormethyl, Trifluormethoxy oder

$C_1$-$C_2$-Alkyl, $C_1$-$C_2$-Alkoxycarbonyl,

sowie deren physiologisch unbedenkliche Salze.

9. Verwendung von Verbindungen der allgemeinen Formel I gemäß Anspruch 1 zur Herstellung von Arzneimitteln zur Bekämpfung von Herzinsuffizienz,

Hypertonie, Herzrhythmusstörungen sowie zur Abschwellung von Schleimhäuten, Senkung des Blutzuckers und Beeinflussung des Salz- und Flüssigkeitshaushaltes.

**Claims**

1. 5-Aryl-1,4-dihydropyridines of the general formula

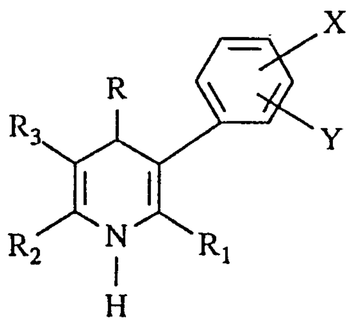

(I)

in which

R represents $C_4$-$C_7$-cycloalkyl,

thienyl, pyridyl, furyl, benzoxadiazolyl, or

phenyl which is optionally substituted by up to three identical or different substituents such as fluorine, chlorine, bromine, nitro, hydroxyl, carboxyl, cyano, $C_1$-$C_4$-alkoxycarbonyl, acetyl, acetyloxy, benzoyl, benzoyloxy, or $C_1$-$C_6$-alkyl, $C_1$-$C_6$-alkoxy, $C_1$-$C_6$-alkylthio or $C_1$-$C_6$-alkylsulphonyl each of which is optionally substituted by one or more fluorine atoms, by phenyl which is optionally substituted by methyl, ethyl, methoxy, fluorine, chlorine or nitro, or optionally by the group -Z-$CH_2$-$R_4$

where

Z represents oxygen or sulphur, and

$R_4$ represents $C_4$-$C_7$-cycloalkyl,

thienyl, furyl or pyridyl, or

phenyl which is optionally substituted by fluorine, chlorine, bromine, cyano, nitro, hydroxyl, carboxyl, $C_1$-$C_4$-alkoxycarbonyl or by $C_1$-$C_6$-alkyl or $C_1$-$C_6$-alkoxy each of which is optionally substituted by one or more fluorine atoms,

$R_1$ and $R_2$ are identical or different and

represent straight-chain or branched $C_1$-$C_5$-alkyl which is optionally substituted by phenyl, carboxyl, hydroxyl or $C_1$-$C_4$-alkoxycarbonyl,

$R_3$ represents nitro, cyano or

the group $-\underset{\underset{O}{\|}}{C}-A-R_5$,

where

A represents oxygen, and where

$R_5$ represents hydrogen,

$C_4$-$C_7$-cycloalkyl, or

a straight-chain or branched, saturated or unsaturated hydrocarbon radical with 1 to 10 C atoms which is optionally interrupted in the chain by one to three oxygen and/or sulphur atoms and is optionally substituted by one or more fluorine, chlorine or bromine atoms, nitro, cyano, hydroxyl, carboxyl, $C_1$-$C_2$-alkoxycarbonyl, phenyl, benzylmethylamino, pyridyl, furyl or thienyl groups

X and Y are identical or different and

represents hydrogen,

$C_1$-$C_4$-alkyl, $C_1$-$C_4$-alkoxy or $C_1$-$C_4$-alkoxycarbonyl, fluorine, chlorine, bromine, hydroxyl, nitro, carboxyl, cyano or

trifluoromethyl, trifluoromethoxy,

and their physiologically acceptable salts.

2. 5-Aryl-1,4-dihydropyridines of the general formula I in claim 1 in which

R represents cyclopentyl or cyclohexyl,

thienyl, furyl or pyridyl, or

phenyl which is optionally substituted by one to two identical or different substituents such as fluorine, chlorine, nitro, carboxyl, $C_1$-$C_2$-alkoxycarbonyl, cyano, phenyl, or $C_1$-$C_4$-alkyl, $C_1$-$C_4$-alkyloxy, $C_1$-$C_4$-alkylthio or $C_1$-$C_4$-alkylsulphonyl each of which is optionally substituted by one or more fluorine atoms, or optionally by the group -Z-$CH_2$-$R_4$,

where

Z represents oxygen or sulphur, and

$R_4$ represents cyclopentyl or cycohexyl,

thienyl, furyl or pyridyl, or

phenyl which is optionally substituted by fluorine, chlorine, cyano, nitro, hydroxyl, carboxyl, $C_1$-$C_2$-alkoxycarbonyl or by $C_1$-$C_4$-alkyl or $C_1$-$C_4$-alkyloxy each of which is optionally substituted by one or more fluorine atoms,

$R_1$ and $R_2$ are identical or different and

represent straight-chain or branched $C_1$-$C_4$-alkyl which is optionally substituted by phenyl, carboxyl or $C_1$-$C_3$-alkoxycarbonyl,

$R_3$ represents nitro or

the group $-\underset{\underset{O}{\|}}{C}-A-R_5$,

where

A represents oxygen,

$R_5$ represents cyclopentyl, cyclohexyl or

a straight-chain or branched, saturated or unsaturated hydrocarbon radical with up 1 to 8 C atoms which is optionally interrupted in the chain by one to two oxygen and/or sulphur atoms and is optionally substituted by one or more fluorine or chlorine atoms, nitro, cyano, hydroxyl or pyridyl groups,

X and Y are identical or different and

represents hydrogen,

fluorine, chlorine, hydroxyl, nitro, cyano,

trifluoromethyl, trifluoromethoxy or

$C_1$-$C_2$-alkyl, $C_1$-$C_2$-alkoxy, $C_1$-$C_2$-alkoxycarbonyl,

and their physiologically acceptable salts.

3. Compounds according to claims 1 to 2, for controlling cardiac insufficiency, hypertension, cardiac arrhythmias, for reducing mucosal swelling, for reducing blood sugar, and for affecting the salt and fluid balance.

4. Medicaments containing as active compound compounds according to claims 1 to 2.

5. Process for the preparation of medicaments according to claim 4, characterised in that the active compound is mixed with customary auxiliaries and excipients.

6. Process for the preparation of 5-aryl-1,4-dihydropyridines of the general formula I

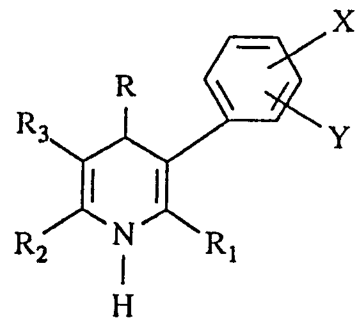

(I)

in which
R represents $C_4$-$C_7$-cycloalkyl,
thienyl, pyridyl, furyl, benzoxadiazolyl, or
phenyl which is optionally substituted by up to three identical or different substituents such as fluorine, chlorine, bromine, nitro, hydroxyl, carboxyl, cyano, $C_1$-$C_4$-alkoxycarbonyl, acetyl, acetyloxy, benzoyl, benzoyloxy, or $C_1$-$C_6$-alkyl, $C_1$-$C_6$-alkoxy, $C_1$-$C_6$-alkylthio or $C_1$-$C_6$-alkylsulphonyl each of which is optionally substituted by one or more fluorine atoms, by phenyl which is optionally substituted by methyl, ethyl, methoxy, fluorine, chlorine or nitro, or optionally by the group $-Z-CH_2-R_4$,
where
Z represents oxygen or sulphur, and
$R_4$ represents $C_4$-$C_7$-cycloalkyl,
thienyl, furyl or pyridyl, or
phenyl which is optionally substituted by fluorine, chlorine, bromine, cyano, nitro, hydroxyl, carboxyl, $C_1$-$C_4$-alkoxycarbonyl or by $C_1$-$C_6$-alkyl or $C_1$-$C_6$-alkoxy each of which is optionally substituted by one or more fluorine atoms,
$R_1$ and $R_2$ are identical or different and
represent straight-chain or branched $C_1$-$C_5$-alkyl which is optionally substituted by phenyl, carboxyl, hydroxyl or $C_1$-$C_4$-alkoxycarbonyl,
$R_3$ represents nitro, cyano or
the group -C-A-$R_5$,
||
O
where
A represents oxygen, and where
$R_5$ represents hydrogen,
$C_4$-$C_7$-cycloalkyl, or
a straight-chain or branched, saturated or unsaturated hydrocarbon radical with 1 to 10 C atoms which is optionally interrupted in the chain by one to three oxygen and/or sulphur atoms and is optionally substituted by one or more fluorine, chlorine or bromine atoms, nitro, cyano, hydroxyl, carboxyl, $C_1$-$C_2$-alkoxycarbonyl, phenyl, benzylmethylamino, pyridyl, furyl or thienyl groups
X and Y are identical or different and
represent hydrogen,
$C_1$-$C_4$-alkyl, $C_1$-$C_4$-alkoxy or $C_1$-$C_4$-alkoxycarbonyl,
fluorine, chlorine, bromine, hydroxyl, nitro, carboxyl, cyano or
trifluoromethyl, trifluoromethoxy,
characterised in that

(a) aldehydes of the general formula II

R
I
CH
||
O

(II)

in which R has the abovementioned meaning, and keto compounds of the general formula III

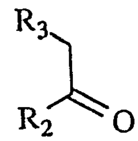

(III)

in which $R_2$ and $R_3$ have the abovementioned meaning, are reacted with keto compounds of the general formula IV

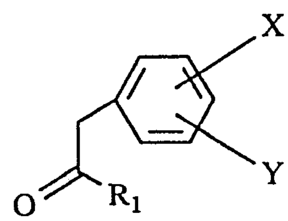

(IV)

in which $R_1$, X and Y have the abovementioned meaning, and ammonia or a suitable ammonium salt, where appropriate in the presence of water or inert organic solvents, or

(b) aldehydes of the general formula II are reacted with keto compounds of the general formula III and enamines, where appropriate prepared in situ, of the general formula V

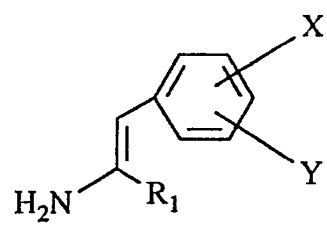

(V)

in which $R_1$, X and Y have the abovementioned meaning, where appropriate in the presence of water or inert organic solvents, or,

(c) aldehydes of the general formula II are reacted with keto compounds of the general formula IV and enamines of the general formue VI

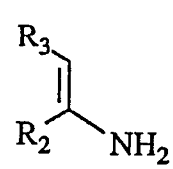

(VI)

in which $R_2$ and $R_3$ have the abovementioned meaning, where appropriate in the presence of water or inert organic solvents, or

(d) ylidene compounds of the general formula VII

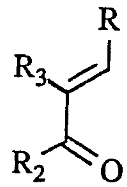

(VII)

in which R, $R_2$ and $R_3$ have the abovementioned meanings, are reacted with keto compounds of the general formula IV and ammonia or a suitable ammonium salt, where appropriate in the presence of water or inert organic solvents, or

(e) keto compounds of the general formula III are reated with ylidene compounds of the general formula VIII

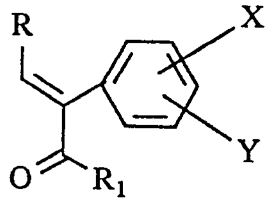

(VIII)

in which R, $R_1$, X and Y have the abovementioned meaning, and ammonia or a suitable ammonium salt, where appropriate in the presence of water or inert organic solvents, or

(f) ylidene compounds of the general formule VII are reacted with enamines of the general formula V, where appropriate in the presente of water or inert organic solvents.

(g) enamines of the general formula VI are reacted, with ylidene compounds of the general formula VIII, where appropriate in the presence of water or inert organic solvents.

7. Process according to claim 6, characterised in that the reactions are carried out at 0 to 200°C.

8. Process according to claims 6 and 7, characterised in that 5-aryl-1,4-dihydropyridines of the general formula I are prepared with the following substituent definitions:

R represents cyclopentyl or cyclohexyl, thienyl, furyl or pyridyl, or phenyl which is optionally substituted by one to two identical or different substituents such as fluorine, chlorine, nitro, carboxyl, $C_1$-$C_2$-alkoxycarbonyl, cyano phenyl, or $C_1$-$C_4$-alkyl, $C_1$-$C_4$-alkyloxy, $C_1$-$C_4$-alkylthio or $C_1$-$C_4$-alkylsulphonyl each of which is optionally substituted by one or more fluorine atoms, or optionally by the group -Z-$CH_2$-$R_4$, where

Z represents oxygen or sulphur, and

$R_4$ represents cyclopentyl or cyclohexyl, thienyl, furyl or pyridyl, or phenyl which is optionally substituted by fluorine, chlorine, cyano, nitro, hydroxyl, carboxyl, $C_1$-$C_2$-alkoxycarbonyl or by $C_1$-$C_4$-alkyl or $C_1$-$C_4$-alkyloxy each of which is optionally substituted by one or more fluorine atoms,

$R_1$ and $R_2$ are identical or different and represent straight-chain or branched $C_1$-$C_4$-alkyl which is optionally substituted by phenyl, carboxyl, hydroxyl or $C_1$-$C_3$-alkoxycarbonyl,

$R_3$ represents nitro or the group -C-A-$R_5$,
‖
O

where

A represents oxygen,

$R_5$ represents cyclopentyl, cyclohexyl or a straight-chain or branched, saturated or unsaturated hydrocarbon radical with up to 8 C atoms which is optionally interrupted in the chain by one to two oxygen and/or sulphur atoms and is optionally substituted by one or more fluorine, chlorine or nitro, cyano, hydroxyl or pyridyl groups,

X and Y are identical or different and represent hydrogen, fluorine, chlorine, hydroxyl, nitro, cyano trifluoromethyl, trifluoromethoxy or $C_1$-$C_2$-alkyl, $C_1$-$C_2$-alkoxy, $C_1$-$C_2$-alkoxycarbonyl, and their physiologically acceptable salts.

9. Use of compounds of the general formula I according to claim 1 for the preparation of medicaments for controlling cardiac insufficiency, hypertension, cardiac arrhythmias, for reducing mucosal swelling, for reducing blood sugar, and for affecting the salt and fluid balance.

**Revendications**

1. 5-aryl-1,4-dihydropyridines de formule générale I

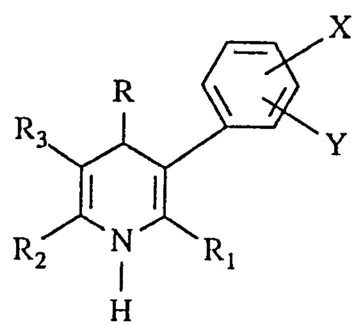

(I)

dans laquelle

R représente un groupe cycloalkyle en $C_4$ à $C_7$ un groupe thiényle, pyridyle, furyle, un groupe benzoxadiazolyle ou

un groupe phényle qui est substitué le cas échéant par jusqu'à trois substituants identiques ou différents tels que fluor, chlore, brome, groupes nitro, hydroxy, carboxy, cyano, (alkoxy en $C_1$ à $C_4$)-carbonyle, acétyle, acétyloxy, benzoyle, benzoyloxy, alkylsulfonyle en $C_1$ à $C_6$, alkylthio en $C_1$ à $C_6$, alkoxy en $C_1$ à $C_6$ ou alkyle en $C_1$ à $C_6$ portant éventuellement comme substituants un ou plusieurs atomes de fluor, par un groupe phényle éventuellement substitué par un radical méthyle, éthyle, méthoxy, fluoro, chloro ou nitro, ou le cas échéant par le groupe -Z-$CH_2$-$R_4$,

dans lequel

Z représente l'oxygène ou le soufre, et

$R_4$ représente un groupe cycloaklyle en $C_4$ à $C_7$
un groupe thiényle, furyle ou pyridyle ou
un groupe phényle qui est substitué le cas échéant par du fluor, du chlore, du brome, un groupe cyano, nitro, hydroxy, carboxy, (alkoxy en $C_1$ à $C_4$)-carbonyle ou par un groupe alkyle en $C_1$ à $C_6$ ou alkoxy en $C_1$ à $C_6$ éventuellement substitué par un ou plusieurs atomes de fluor
$R_1$, $R_2$ sont identiques ou différents et représentent
un groupe alkyle en $C_1$ à $C_5$ à chaîne droite ou à chaîne ramifiée, qui est substitué le cas échéant par un radical phényle, carboxy, hydroxy ou (alkoxy en $C_1$ à $C_4$)-carbonyle
$R_3$ est un groupe nitro, cyano ou
le groupe -C-A-$R_5$
||
O
dans lequel
A désigne l'oxygène et
$R_5$ représente l'hydrogène,
un groupe cycloalkyle en $C_4$ à $C_7$ ou
un reste hydrocarboné de 1 à 10 atomes de carbone à chaîne droite ou à chaîne ramifiée, saturé ou non saturé, à chaîne éventuellement interrompue par 1 à 3 atomes d'oxygène et/ou de soufre, qui est substitué le cas échéant par un ou plusieurs atomes de fluor, de chlore, de brome ou groupes nitro, cyano, hydroxy, carboxy, (alkoxy en $C_1$ ou $C_2$)-carbonyle, phényle, benzylméthylamino, pyridyle, furyle ou thiényle,
X, Y sont égaux ou différents et représentent l'hydrogène,
un groupe alkyle en $C_1$ à $C_4$, alkoxy en $C_1$ à $C_4$ ou (alkoxy en $C_1$ à $C_4$)-carbonyle
le fluor, le chlore, le brome, un groupe hydroxy, nitro, carboxy, cyano ou
un groupe trifluorométhyle, trifluorométhoxy, ainsi que leurs sels acceptables du point de vue physiologique.

2. 5-aryl-1,4-dihydropyridines de formule générale I suivant la revendication 1, dans laquelle
R représente un groupe cyclopentyle ou cyclohexyle,
un groupe thiényle, furyle ou pyridyle, ou
un groupe phényle, qui est substitué le cas échéant par un ou deux substituants identiques ou différents tels que fluor, chlore, nitro, carboxy, (alkoxy en $C_1$ ou $C_2$)-carbonyle, cyano, phényle, alkylsulfonyle en $C_1$ à $C_4$, alkylthio en $C_1$ à $C_4$, alkyloxy en $C_1$ à $C_4$, alkyle en $C_1$ à $C_4$ portant éventuellement comme substituants un ou plusieurs atomes de fluor, ou le cas échéant par le groupe -Z-$CH_2$-$R_4$,
dans lequel
Z représente l'oxygène ou le soufre et
$R_4$ représente un groupe cyclopentyle ou cyclohexyle,
un groupe thiényle, furyle ou pyridyle ou
un groupe phényle qui est substitué le cas échéant par du fluor, du chlore, un groupe cyano, nitro, hydroxy, carboxy, (alkoxy en $C_1$ ou $C_2$)-carbonyle ou par un groupe alkyle en $C_1$ à $C_4$ ou alkyloxy en $C_1$ à $C_4$ portant éventuellement comme substituants un ou plusieurs atomes de fluor,
$R_1$, $R_2$ sont identiques ou différents et représentent
un groupe alkyle en $C_1$ à $C_4$ à chaîne droite ou à chaîne ramifiée qui est substitué le cas échéant par un radical phényle, carboxy ou (alkoxy en $C_1$ à $C_3$)-carbonyle
$R_3$ représente un groupe nitro ou
le groupe -C-A-$R_5$
||
O
dans lequel
A est l'oxygène,
$R_5$ représente un groupe cyclopentyle, cyclohexyle ou
un reste d'hydrocarbure ayant jusqu'à huit atomes de carbone à chaîne droite ou ramifiée, saturé ou non saturé, dont la chaîne est interrompue le cas échéant par un ou deux atomes d'oxygène et/ou de soufre, qui est éventuellement substitué par un ou plusieurs atomes de fluor ou de chlore ou groupes nitro, cyano, hydroxy ou pyridyle,
X, Y sont identiques ou différents et représentent l'hydrogène
le fluor, le chlore, un groupe hydroxy, nitro, cyano,
un groupe trifluorométhyle, trifluorométhoxy, ou
un groupe alkyle en $C_1$ ou $C_2$, alkoxy en $C_1$ ou $C_2$, (alkoxy en $C_1$ ou $C_2$)-carbonyle,
ainsi que leurs sels acceptables du point de vue physiologique.

3. Composés suivant les revendications 1 et 2, destinés à combattre des insuffisances cardiaques, l'hypertonie, des perturbations du rythme cardiaque ainsi que pour décongestionner les muqueuses, pour abaisser la glycémie et pour influencer le bilan salin et le bilan en liquide.

4. Médicament contenant comme substance active des composés suivant les revendications 1 et 2.

5. Procédé de préparation de médicaments suivant la revendication 4, caractérisé en ce qu'on mélange la substance active avec des adjuvants et des supports classiques.

6. Procédé de préparation de 5-aryl-1,4-dihydropyridines de formule générale I

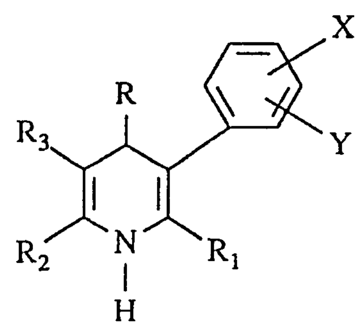

(I)

dans laquelle
R représente un groupe cycloalkyle en $C_4$ à $C_7$
un groupe thiényle, pyridyle, furyle, un groupe benzoxadiazolyle ou
un groupe phényle qui est substitué le cas échéant par jusqu'à trois substituants identiques ou différents tels que fluor, chlore, brome, groupes nitro,

hydroxy, carboxy, cyano, (alkoxy en $C_1$ à $C_4$)-carbonyle, acétyle, acétyloxy, benzoyle, benzoyloxy, alkylsulfonyle en $C_1$ à $C_6$, alkylthio en $C_1$ à $C_6$, alkoxy en $C_1$ à $C_6$ ou alkyle en $C_1$ à $C_6$ portant éventuellement comme substituants un ou plusieurs atomes de fluor, par un groupe phényle éventuellement substitué par un radical méthyle, éthyle, méthoxy, fluoro, chloro ou nitro, ou le cas échéant par le groupe $-Z-CH_2-R_4$,

dans lequel

Z représente l'oxygène ou le soufre, et

$R_4$ représente un groupe cycloaklyle en $C_4$ à $C_7$

un groupe thiényle, furyle ou pyridyle ou

un groupe phényle qui est substitué le cas échéant par du fluor, du chlore, du brome, un groupe cyano, nitro, hydroxy, carboxy, (alkoxy en $C_1$ à $C_4$)-carbonyle ou par un groupe alkyle en $C_1$ à $C_6$ ou alkoxy en $C_1$ à $C_6$ éventuellement substitué par un ou plusieurs atomes de fluor

$R_1$, $R_2$ sont identiques ou différents et représentent

un groupe alkyle en $C_1$ à $C_5$ à chaîne droite ou à chaîne ramifiée, qui est substitué le cas échéant par un radical phényle, carboxy, hydroxy ou (alkoxy en $C_1$ à $C_4$)-carbonyle

$R_3$ est un groupe nitro, cyano ou

le groupe -C-A-$R_5$
‖
O

dans lequel

A désigne l'oxygène et

$R_5$ représente l'hydrogène,

un groupe cycloalkyle en $C_4$ à $C_7$ ou

un reste hydrocarboné de 1 à 10 atomes de carbone à chaîne droite ou à chaîne ramifiée, saturé ou non saturé, à chaîne éventuellement interrompue par 1 à 3 atomes d'oxygène et/ou de soufre, qui est substitué le cas échéant par un ou plusieurs atomes de fluor, de chlore, de brome ou groupes nitro, cyano, hydroxy, carboxy, (alkoxy en $C_1$ ou $C_2$)-carbonyle, phényle, benzylméthylamino, pyridyle, furyle ou thiényle,

X, Y sont identiques ou différents et représentent l'hydrogène,

un groupe alkyle en $C_1$ à $C_4$, alkoxy en $C_1$ à $C_4$ ou (alkoxy en $C_1$ à $C_4$)-carbonyle

le fluor, le chlore, le brome, un groupe hydroxy, nitro, carboxy, cyano ou

un groupe trifluorométhyle, trifluorométhoxy, caractérisé en ce que

(a) on fait réagir des aldéhydes de formule générale II

R
|
CH (II)
‖
O

dans laquelle R a la définition indiquée ci-dessus et des composés cétoniques de formule générale III

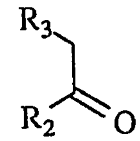
(III)

dans laquelle $R_2$, $R_3$ ont les définitions indiquées ci-dessus, avec des composés cétoniques de formule générale IV

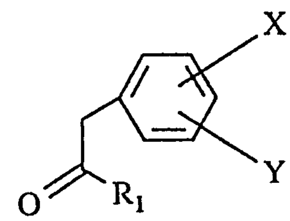
(IV)

dans laquelle $R_1$, X, Y ont la définition indiquée ci-dessus, et l'ammoniac ou un sel d'ammonium approprié, le cas échéant en présence d'eau ou de solvants organiques inertes,

ou bien

(b) on fait réagir des aldéhydes de formule générale II avec des composés cétoniques de formule générale III et des énamines — préparées le cas échéant in situ — de formule générale V

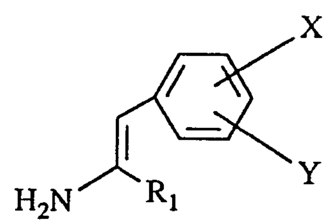
(V)

dans laquelle $R_1$, X, Y ont la définition indiquée ci-dessus, le cas échéant en présence d'eau ou de solvants organiques inertes,

ou bien

(c) on fait réagir des aldéhydes de formule générale II avec des composés cétoniques de formule générale IV et des énamines de formule générale VI

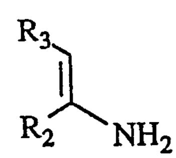
(VI)

dans laquelle $R_2$, $R_3$ ont la définition indiquée ci-dessus, le cas échéant en présence d'eau ou de solvants organiques inertes,

ou bien

(d) on fait réagir des composés ylidéniques de formule générale VII

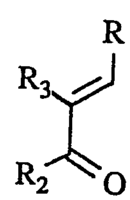
(VII)

dans laquelle
R, $R_2$, $R_3$ ont les définitions indiquées ci-dessus, avec des composés cétoniques de formule générale IV et l'ammoniac ou un sel d'ammonium approprié, le cas échéant en présence d'eau ou de solvants organiques inertes,
ou bien
(e) on fait réagir des composés cétoniques de formule générale III avec des composés ylidéniques de formule générale VIII

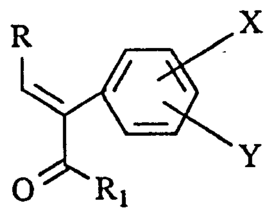

(VIII)

dans laquelle
R, $R_1$, X, Y ont la défnition indiquée ci-dessus, et l'ammoniac ou un sel d'ammonium approprié, le cas échéant en présence d'eau ou de solvants organiques inertes,
ou bien
(f) on fait réagir des composés ylidéniques de formule générale VII avec des énamines de formule générale V, le cas échéant en présence d'eau ou de solvants organiques inertes
ou bien
(g) on fait réagir des énamines de formule générale VI avec des composés ylidéniques de formule générale VIII, éventuellement en présence d'eau ou de solvants organiques inertes.

7. Procédé suivant la revendication 6, caractérisé en ce qu'on conduit les réactions à une température de 0 à 200°C.

8. Procédé suivant les revendications 6 et 7, caractérisé en ce qu'on prépare des 5-aryl-1,4-dihydropyridines de formule générale I dont les substituants ont les définitions suivantes:
R représente un groupe cyclopentyle ou cyclohexyle,
un groupe thiényle, furyle ou pyridyle, ou
un groupe phényle, qui est substitué le cas échéant par un ou deux substituants identiques ou différents tels que fluor, chlore, nitro, carboxy, (alkoxy en $C_1$ ou $C_2$)-carbonyle, cyano, phényle, alkylsulfonyle en $C_1$ à $C_4$, alkylthio en $C_1$ à $C_4$, alkyoxy en $C_1$ à $C_4$, alkyle en $C_1$ à $C_4$ portant éventuellement comme substituants un ou plusieurs atomes de fluor, ou le cas échéant par le groupe $-Z-CH_2-R_4$,
dans lequel
Z représente l'oxygène ou le soufre et
$R_4$ représente un groupe cyclopentyle ou cyclohexyle,
un groupe thiényle, furyle ou pyridyle ou
un groupe phényle qui est substitué le cas échéant par du fluor, du chlore, un groupe cyano, nitro, hydroxy, carboxy, (alkoxy en $C_1$ ou $C_2$)-carbonyle ou par un groupe alkyle en $C_1$ à $C_4$ ou alkyloxy en $C_1$ à $C_4$ portant éventuellement comme substituants un ou plusieurs atomes de fluor,
$R_1$, $R_2$ sont identiques ou différents et représentent
un groupe alkyle en $C_1$ à $C_4$ à chaîne droite ou à chaîne ramifiée qui est substitué le cas échéant par un radical phényle, carboxy ou (alkoxy en $C_1$ à $C_3$)-carbonyle,
$R_3$ représente un groupe nitro ou
le groupe $-\underset{\underset{O}{\|}}{C}-A-R_5$
dans lequel
A est l'oxygène,
$R_5$ représente un groupe cyclopentyle, cyclohexyle ou
un reste d'hydrocarbure ayant jusqu'à huit atomes de carbone à chaîne droite ou ramifiée, saturé ou non saturé, dont la chaîne est interrompue le cas échéant par un ou deux atomes d'oxygène et/ou de soufre, qui est éventuellement substitué par un ou plusieurs atomes de fluor ou de chlore ou groupes nitro, cyano, hydroxy ou pyridyle
X, Y sont identiques ou différents et représentent
l'hydrogène
le fluor, le chlore, un groupe hydroxy, nitro, cyano
un groupe trifluorométhyle, trifluorométhoxy ou
un groupe alkyle en $C_1$ ou $C_2$, alkoxy en $C_1$ ou $C_2$, (alkoxy en $C_1$ ou $C_2$)-carbonyle,
ainsi que leurs sels physiologiquement acceptables.

9. Utilisation de composés de formule générale I suivant la revendication 1 pour la préparation de médicaments destinés à combattre l'insuffisance cardiaque, l'hypertonie, les troubles du rythme cardiaque ainsi que pour décongestionner les muqueuses, pour abaisser le taux de glycémie et pour influencer le bilan salin et le bilan en liquide.